# EUROPEAN PATENT SPECIFICATION

(11) **EP 2 883 950 B1**
(45) Date of publication and mention of the grant of the patent: **08.04.2020**
(21) Application number: 13196793.7
(22) Date of filing: 12.12.2013
(51) Int. Cl.: C12M 1/00

(54) **Light system for aquatic photosynthetic organisms**
Lichtsystem für photosynthetische Wasserorganismen
Système d'éclairage pour des organismes photosynthétiques aquatiques

(43) Date of publication of application: 17.06.2015
(73) Proprietor: Stazione Zoologica "Anton Dohrn", 80121 Napoli (IT)
(72) Inventor: Brunet, Christophe, 80121 Napoli (NA) (IT); Corato, Federico, 80121 Napoli (NA) (IT)
(74) Representative: Capasso, Olga

(56) References cited:
- CN-U- 201 746 536
- CN-U- 202 072 705
- US-A1- 2005 135 104
- CHENG YAN ET AL: "Performance of purifying anaerobic fermentation slurry using microalgae in response to various LED light wavelengths and intensities", JOURNAL OF CHEMICAL TECHNOLOGY & BIOTECHNOLOGY, vol. 88, no. 9, 28 September 2013 (2013-09-28), pages 1622-1630, XP055120583, ISSN: 0268-2575, DOI: 10.1002/jctb.4010

## Description

### TECHNICAL FIELD

The present invention relates to a light system for aquatic photosynthetic organisms, which is based on LED sensors technology. The present system is able to adjust light intensity and quality emitted by LED sensors, in such a way to generate a light very similar to natural sea light. The system can reproduce every natural light of the aquatic ecosystems and can be widely and finely tuned by the user.

### BACKGROUND ART

In nature, light is the most important and variable ecological parameter to ensure photosynthetic organisms growth. In fact, light is responsible for the photosynthetic process, transforming light energy in biochemical energy.

Therefore, reproducing natural sea light by means of an artificial light system represents a crucial issue for experiments and growth of all aquatic photosynthetic organisms, in particular microalgae. However reproducing natural sea light is difficult due to the optical properties of sea water and to different aspects related to light penetration in water column. Indeed, light intensity and spectrum are a function of water depth, season, latitude, hour of the day, sky brightness and so on. Moreover, in coastal ecosystems, microalgae are subject to fast variations of both light intensity and spectrum during the daytime since they grow in a water column submitted to mixing due to wind and/or currents.

In general, upper layers of aquatic systems and pelagic lakes are characterized by high light intensity, whose spectrum is substantially composed of blue, green and red color. On the other hand, in deeper oceans only blue spectrum is present. In coastal ecosystems, rich in organic and mineral substances, light intensity is decreased and the intensity ratio among blue, green and red changes relatively to the previous ecosystems.

Experimental studies on microalgae have been shown that light has a primordial role in varying the ecophysiological state of cells, modifying the capacity of photosynthesis and growth. However, none of the available illumination systems to grow microalgae allow to reproduce or simulate the natural sea light, both in term of quality and temporal distribution.

Therefore there is a need for a light system well adapted for aquatic photosynthetic organisms growth, which takes into account the above mentioned parameters and is able to reproduce a light comparable to the natural sea light.

Algal growth can be done "outdoor" in large tanks. The limits of such a system are many: the spectrum of the light is that of air, different from the marine environment. The cost of maintenance is high for example due to heating of the tanks in the brightest seasons and the risk of evaporation, which leads to increase the salinity of the water. Finally, this system is not versatile, not allowing to vary any key light parameter.

"Indoor" light systems are of different types. On large scale, they can use the solar light when placed in a "greenhouse" which reduces cost. However, the light is far from being the one from marine environment.

Systems which use artificial light are composed of "neon ", discharge lamps or LEDs . Limits from using neons are that (i) the emitted light spectrum is different from that of the marine environment (high presence of red, for example), (ii) the light intensity is relatively low, (iii) neon systems are very bulky, (iv) the energy consumption is medium and the heat supplied by the light is quite high, and (v) generally, light intensity from neons is not adjustable since they often allow only an "on" / "off" position. Moreover, when the "neons systems" are adjustable, the lamp life is reduced which increases the cost, and the light spectrum changes depending on the intensity (due to circulating gaz in the neon tubes). This system is often used in so-called "photobioreactors" systems of algal mass growth.

Other systems use discharge lamps. For example, the systems used for aquarium. Their spectra may be more appropriate to the deep marine ecosystem (predominantly blue, then adapted for example to the growth of corals). The limits of these lamps and systems are: heating of the lamps and the environment, fixed spectrum, impossibility to vary the frequency of light emission as desired, high energy consumption, limited duration of life of the lamp and its cost. These systems therefore are not versatile. Finally, some LED systems exist but they are of low light intensity. These systems are mono-spectral or integrate some blue and red light, but the green component (which makes the light appear "white" to the human eye) is generally missing. When the three blue, red and green colors are present all together, such system does not allow to vary the colors separately, with the same device. Then there is no system which manages three " spectra " of light, in terms of frequency variability, intensity, and the contribution of each color.

E.g. US 2005/135104 refers to a lighting system for photobioreactors suitable for culture of phototrophic microorganisms. Up to now, an artificial light system able to perfectly reproduce every kind of natural light of aquatic ecosystems is unknown. Furthermore, a light system that is simple, as well as very precise and programmable in real time is also unknown. The present invention provides a new system able to reproduce every light condition, which is suitable and fundamental for algal growth, maintenance of algal culture, mass production of algal biomass as well as for experimental tests on aquatic algae.

### SUMMARY OF THE INVENTION

The present invention is a light system, utilizing light emitting diode (LED), particularly the species of Z- POWER LED-RGB, which substantially provide a three color light: red, green and blue. Moreover, by combining such colors, a wide range of visible light spectrum (400-700 nm) can be obtained. For instance, it is possible to obtain white light by mixing the three colors at their maximum intensity.

This system allows, by varying the quality and the intensity of light on time scales that goes from seconds till hours (for instance the diel cycle of 24 hours), to perform circadian cycles, according to a sinusoidal light distribution as the solar light does.

Vertical spectrum profiles of light, measured at different stations in the Mediterranean Sea, have been examined to conceive the present system, with the aim to reproduce different natural light conditions in the different aquatic environments.

The system of the present invention allows to reproduce any kind of natural light in the aquatic ecosystems and it can be set up as desired by the user. Latitude and seasons can be simulated through the variations of photoperiod set-up as well as the daily-integrated light and maximum intensity peak (solar mid-day). The system of the present invention perfectly simulates the three main "bio-optical" aquatic ecosystems: the upper layer of pelagic systems and lakes, deeper oceans and coastal ecosystems. With the system of the present invention it is also possible to set up the lightening gradient from the top to the bottom of the water column.

The system, being composed by LEDs, is plane, light, easily transportable and suitable to light surface of different sizes. In order to obtain the best performance in terms of light intensity, the lightened surface has to be plane and not round.

Moreover, the present system is advantageous in that it allows to obtain a high intensity light, comparable to the one present on the sea surface. Indeed the system of the present invention is composed of LEDs that do not increase the temperature of illuminated environment and therefore can be positioned close (less than 1 meter, preferably less than 10 cm, still preferably less than 5 or 1 cm) to the surface to be illuminated, allowing to reach high light intensities. Moreover, the LEDs, not generating any heat dissipation, allow to keep the culture flask at constant temperature.

The system can be applied sideways, according to the user' necessities, for instance in case of need to reproduce homogeneous light all over the surface. If, instead, there is the necessity to reproduce a vertical light extinction, as occurring in the water mass, the present system may be placed on top of the culture flask. This "top position" will be also preferred for aquarium illumination to avoid eyesight problems for the observer that should occur if the illumination system should be behind the culture flask (for instance, aquarium).

Another fundamental and innovative aspect of the present system is its extremely low energy consumption, due to the presence of LEDs. Moreover, the development of a solar energy system, associated to the system, allows to further reduce the production cost.

In addition, the present system is versatile, modular and occupies a small space, because it is flat (1-2 cm thick).

Being modular, the present system allows to cover a wide range of surfaces, ranging from small size space such as the ones used for experiments on small culture flasks or small acquariums to the large size space for large-scale production of algal biomass. For the latter aspect, the system is well adapted for covering walls for instance to illuminate very large volume of water.

The present invention provides a light system and a method of controlling the same, the method being implementable in a suitable software. The method allows to adjust and modify, according to the needs, the light intensity in the visible spectrum (which is useful for photosynthesis), the intensity and the percentage of each color, red, green, blue (RGB), the time based variability (which is fundamental for the well-being of algae in culture and thus for growth, being the pelagic system under continuous movement), the illumination time and light distribution during the day time.

The system is managed by a software, which allows to change:
* The light intensity in the visible spectrum (the one useful for photosynthesis),
* The intensity and the contribution of each color, Red, Green and Blue (RGB)
* The frequency of temporal variability, which is a parameter of fundamental importance for algal growth (being the pelagic systems in continuous motion),
* The lighting time during the 24 hours of the day and the distribution of light during the day.

All these aspects make possible to reproduce the aquatic light environment for any season and pelagic ecosystem (the waters of the lake, coastal, estuary, deep systems, etc.).

Because of all the innovative aspects of the system, e.g. sinusoidal light distribution together with spectral variations, not only it is possible to maintain algal growth but also to increase the production of molecules such as antioxidants.

It is therefore an object of the present invention a light system (1) for aquatic photosynthetic organisms comprising:
- at least one panel (2), having a plurality of light emitting diode, a LED, sensors, said LED sensors being independent of each other and being RGB LED sensors for providing a three color light comprising blue, red and green;
- three microchips coupled to the LED sensors for modifying the light color emitted by each LED sensor;
- an electronic device (3), said electronic device being connected to said at least one panel (2); and
- a computer (4)
said light system being characterized in that it is configured to combine the three colors with each other to obtain a wide range of different colors in the visible spectrum and wherein the light intensity of each color is independently between 0 and 600 µmol.m⁻² .s⁻¹. Preferably the light intensity of each color is independently modulated or controlled to be between 0 and 600 µmol.m⁻² s⁻¹.

Preferably, the size of said panel (2) has the same size as the surface to be illuminated. Then the panels may be square or rectangular, or nay suitable shape.

Still preferably, the size of said panel (2) ranges between 10 cm to 100 m in length and/or in height.

Preferably, each panel (2) provides a light intensity reaching a maximal of 1800 µmol.m⁻².s⁻¹. Still preferably, the light shape distribution is regulated at very short time scale (less than 1 min, less than 30s or less than 1 s), so that, it can be sinusoidal with high frequency time variations of light intensity and spectral composition ranging from seconds till hours to perform circadian cycles, or of any shape decided by the user. Yet preferably the light intensity and/or light color may be varied in time by the user.

In a preferred embodiment said electronic device (3) comprises:
- a microprocessor (5), which interacts with the computer (4);
- an "EEPROM card" (6) for storing the data used for the setting of the system;
- an optoisolator (7) that allows to transfer a signal between two circuits while maintaining isolation between them using an optical system;
- an output circuit (8) with at least one transistor and
- a power supply board (9).

It is a further object of the invention a bioreactor comprising the light system of the invention.

It is a further object of the invention the use of the light system or of the bioreactor of the invention for the production of biomass, for the production of biomolecules, for the production of biofuel and/or biogas.

It is a further object of the invention the use of the light system or of the bioreactor of the invention in a bioremediation method.

It is a further object of the invention a method of controlling in real time the light system of the invention, characterized by the following steps:
a. initializing system parameters;
b. determining a photoperiod (light period vs. dark period);
c. sending of the signal to the light system;
d. light setting;
e. controlling the correct light setting (if ok, continue to step f, else go back to step b);
f. enabling a time counter to execute step g;
g. reading the light setting file;
h. controlling the time counter (if overcomes a maximum threshold continue to step i, else go back to step g)
i. checking execution and control steps (if ok continue to step j, else go back to step b);
j. ending the program.

It is a further object of the invention a computer program comprising a computer-code suitable for performing the method as described above.

It is a further object of the invention a computer program product on which the computer program as defined above is stored.

In the present system any LED sensor may be used. Preferably the LED has a diameter ranging between 1.0 mm and 5.0 mm. Preferably the system weighs less than 2 kg (depending on the size). More preferably the system is flat and light.

In the present invention a bioreactor or photobioreactor is a mass culture system for aquatic organisms, such as algae, that is transparent, of large volume (10 to hundreds of liters) that contains culture media and appropriated nutrients to favour cell growth. In the present system the LED sensors provide for each color independently a light intensity between 0 and 600 µmol.m⁻².s⁻¹.

Different non limiting embodiments of the invention will be hereafter described, by means of non limiting examples, with reference to the enclosed drawings, where:
Figure 1 shows a scheme of an example of the whole light system according to the present invention. The light system is composed by three independent units, all three managed by the same computer and program.
Figure 2 shows a scheme of the LED light fluxes, according to an embodiment of the present invention.
Figure 3 shows a block diagram of the A/D interface, according to another embodiment of the present invention.
Figure 4 is a graph showing the light intensity distribution, during the day time, in a first period of the year and at a predetermined depth. Unit: light intensity in µmol.m⁻².s⁻¹.
Time corresponds to the hours of the day. Example of light distribution during the day provided by the present system. Note that on these plots, the light color is not represented, but it is possible to manipulate, as desired the spectrum of the emitted light. That means that light can be only blue, red or green or a mixture of two or three colors.

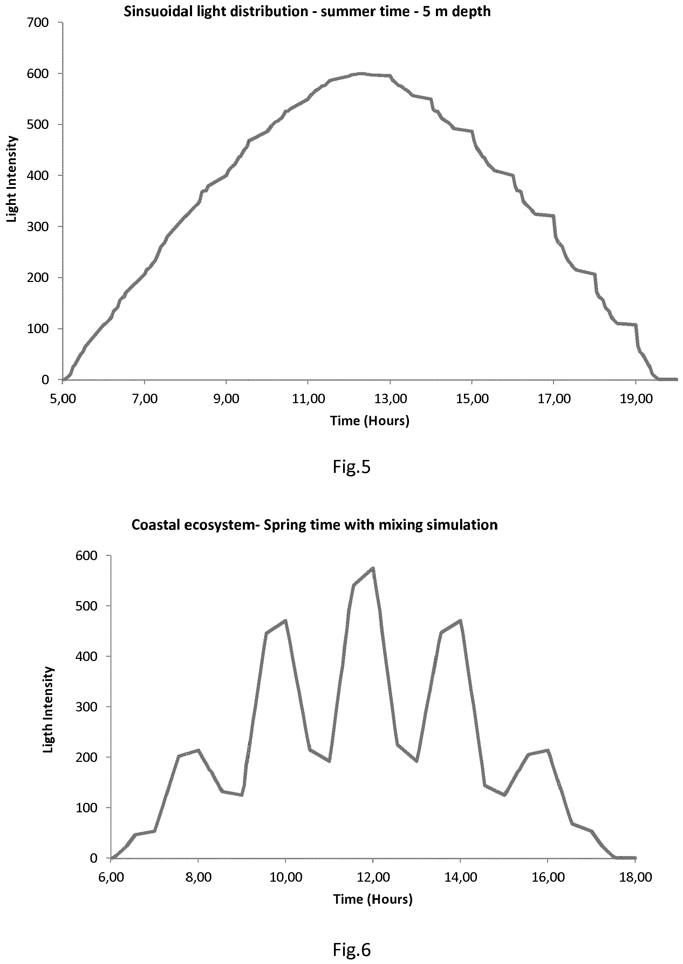

Figure 5 is a graph showing the light intensity distribution, during the day time, in a second period of the year and at a predetermined depth. Unit: light intensity in µmol.m⁻².s⁻¹. Time corresponds to the hours of the day. Example of light distribution during the day provided by the present system. Note that on these plots, the light color is not represented, but it is possible to manipulate, as desired the spectrum of the emitted light. That means that light can be only blue, red or green or a mixture of two or three colors.
Figure 6 is a graph showing the light intensity distribution, during the day time, in a third period of the year and at a predetermined depth. Unit: light intensity in µmol.m⁻².s⁻¹. Time corresponds to the hours of the day. Example of light distribution during the day provided by the present system. Note that on these plots, the light color is not represented, but it is possible to manipulate, as desired the spectrum of the emitted light. That means that light can be only blue, red or green or a mixture of two or three colors.

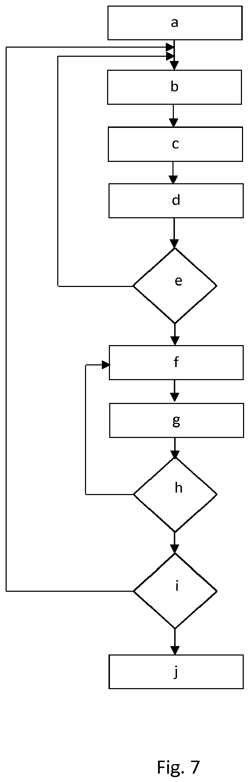

Figure 7 shows the flow-chart of the method of controlling the light system, according to the present invention.

### DETAILED DESCRIPTION OF THE INVENTION

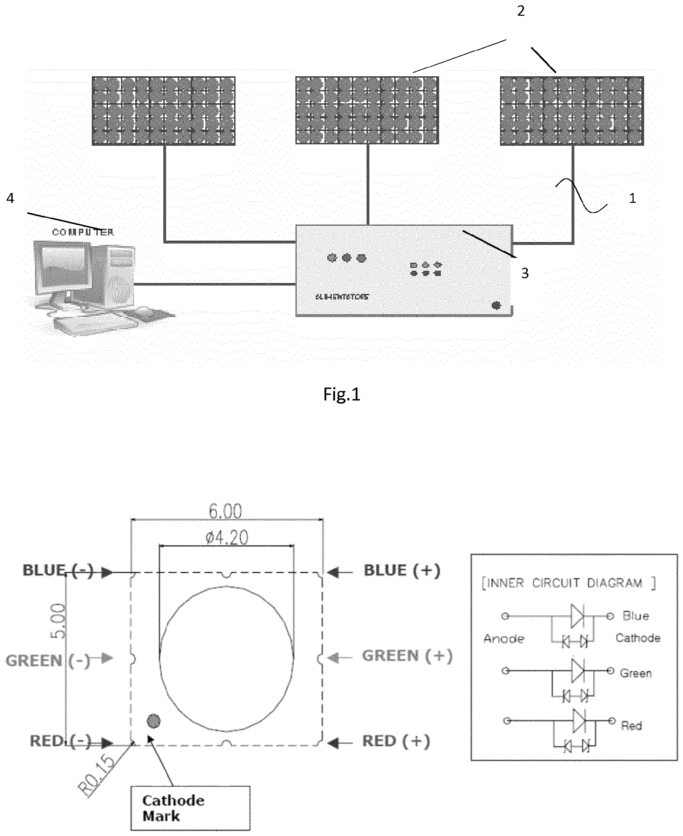

With reference to Fig.1, the light system according to an embodiment of the present invention comprises one or more panels (2) (in the example of Fig. 1, the panels are in number of three) each of them provided with a plurality of LEDs, which are independent from each other and connected to an electronic device (3). Said electronic device is managed by a computer (4), in which a software, implementing a method of controlling the light system, can run.

The software has been written in a BASIC language and can be utilized with all computers. The electronic device is an analogic/digital (A/D) interface and is connected through a serial cable or USB adapted.

According to a preferred embodiment, the size each panel (2) of the light system ranges between 40 - 50 cm in length and between 15-20 cm in height (for example, the panel can be equal to 45 cm in length x 18,5 cm in height, in other words can have a surface equal to 830 cm²). Each panel has 60 Z- Power LED, with maximal light intensity of 1600 µmol.m⁻².s⁻¹, measured with a PARmeter (QSL 2101, Biospherical Instruments Inc., San Diego, CA, USA) at 2 cm from the light source system. The lightened area is of 700 cm² with an illuminated water volume of 5000 ml.

As shown in Fig.2, the LED diodes have small size, the LED diameter ranging between 4.5 mm and 5 mm (for example, in Fig. 2 the diameter is equal to 4.2 mm) and are able to create light effects by mixing red, green and blue colors. The photon flux is equal to 13 lm for blue light, 35 lm for red light and 57 lm for green light. Mixing the three colors, red, blue and green, it is possible to create specific light effects, dependent on the intensity of each of these three colors. The LED sensors work with three microchips mounted in the same "box", that allow to modify the light color emitted by each LED. To mix the colors, a method of controlling the system is used, by means of the A/D interface, driven by the computer.

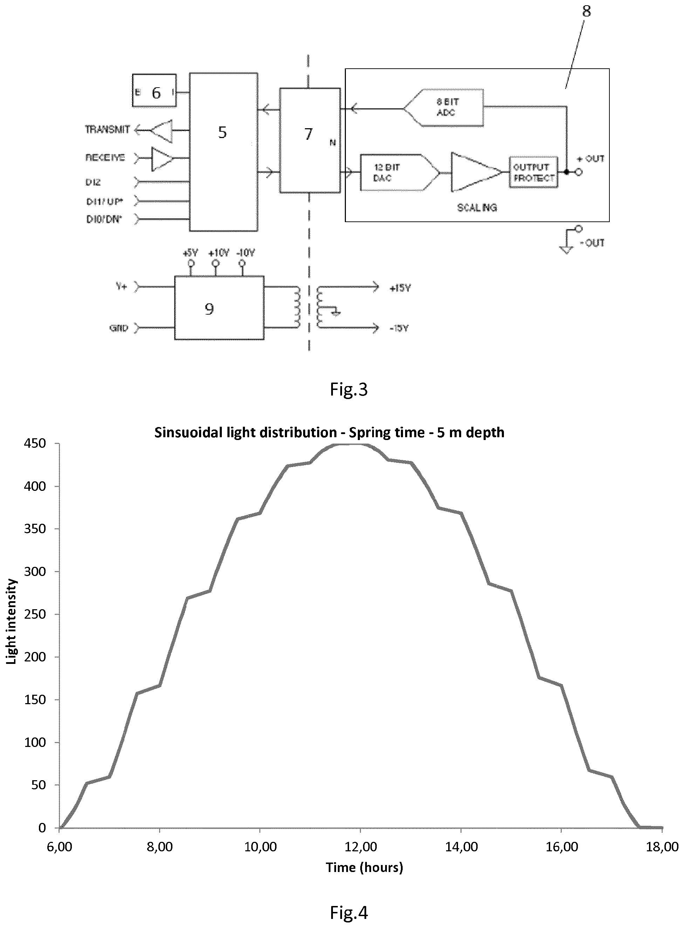

Fig.3 represents a block diagram of the A/D interface, which is used in the present light system. The interface comprises a microprocessor (5) that communicates with the computer through a software, an "EEPROM card" (6) for storing the data used for the setting of the system, an optoisolator (7) that allows to transfer a signal between two circuits while maintaining isolation between them using an optical system, an output circuit (8) with transistor and a power supply board (9). The digital-analogic converter is a device capable of converting a digital signal into an analogic signal.

Figs. 4, 5 and 6 are graphs showing the light intensity distribution, during the day time, light distribution obtained by the present light system. The light color is not represented, but for each case, it is possible to manipulate, as desired, the spectrum of the emitted light. This means that light can be only blue, red or green or a mixture of two or three colors.

The graph in Fig.4 represents the light distribution at 5 m depth during spring-time in a Mediterranean coastal site. The photoperiod is setup at 12 hours of light and 12 hours of dark, the maximum intensity is about 450 µmol.m⁻².s⁻¹, and the light is composed by 40 % blue, 40% green and 20% red color.

The graph in Fig.5 represents the light distribution at 5 m depth during summer-time in a Mediterranean coastal site. The analysed photoperiod is 15 hours of light and 9 hours of dark, the maximum intensity is about 600 µmol.m⁻².s⁻¹, and the light is composed by 50 % blue, 30% green and 20% red color.

The graph in Fig.6 represents the light experienced by microalgae during spring-time in a Mediterranean coastal site, submitted to high mixing. Mixing is setup with light values recorded between 2 and 20 meters depth. The analysed photoperiod is 12 hours of light and 12 hours of dark, the maximum intensity is about 600 µmol.m⁻².s⁻¹, and the light at the surface (light peaks) is composed by 40 % blue, 40% green and 20% red color, while during low light periods, light is composed by 50 % blue, 45% green and 5 % red color.

The method of controlling the light system, whose flow chart is shown in Fig.7, comprises the following steps:
a. initializing system parameters;
b. determining a photoperiod (light period vs. dark period);
c. sending of the signal to the light system;
d. opening light setting file
e. controlling the correct light setting (if ok, continue to step f, else go back to step b);
f. enabling a time counter to execute step g;
g. reading the light setting file;
h. controlling the time counter (if overcomes a maximum threshold continue to step i, else go back to step g)
i. checking execution and control steps (if ok continue to step j, else go back to step b);
j. ending the program.

The method of controlling the light system of the present invention allows to program, in real time, the variation of light intensity during the day. High frequency time variations (minutes to hours) of light intensity and spectral composition can be integrated on the sinusoidal distribution of lightening phase. This innovative function allows reproducing the light fluctuations experienced by microalgae, due to water column mixing by currents, wind or any vertical advection. This function is relevant in a perspective of more realistic simulation of pelagic systems.

Studies conducted with less performing light systems (monospectral light) (Dimier et al., 2009a,b; Corato et al., 2007) have shown that high frequency time variations of light intensity allow to gain microalgal biomass yield.

The use of our new light system further increases the yield of aquatic organisms thanks to the coupling of light spectrum with its intensity and the possibility to make any kind of combination between them.

The applications of the light system are several: i) aquariology, both for professional (catering, tourism, etc.) and family fish tanks, ii) marine organisms research, for marine organisms maintained, both in public and private institutions, iii) biotechnological applications, for biomass production from aquatic plants.

In particular, the present light system can find application in several biotechnological fields (environmental, energetics, pharmaceutics, nutraceutics, cosmetics). Moreover, the light system is particularly appropriate for the so-called "photosynthetic biotechnology" because of its efficiency for algal growth and biomolecules production, thanks to the applied variations of light conditions. In detail:
- in cosmetics, for production and use of specific biomolecules as carotenoids, polyunsaturated fatty acids (PUFA), phycobiliproteins and antioxidants;
- in pharmaceutics, for isolation of effective biomolecules against illness or for human health defense.
- in nutraceutics, for human and animal feeding formulations (aquaculture) with high content of antioxidant molecules (carotenoids), high nutritional value proteins, microelements and polyunsaturated fatty acids;
- in research field, for development of fluorescent labeling molecules, as phycobiliproteins;
- in green energy field, for biogas and biofuels production due to the high lipid content of several species of microalgae;
- in environmental field, for CO₂ (linked to biogas production) and NOx (scraps from thermoelectric factories or other industrial systems) recycling;
- in waste water treatments, through the use of algae, which let nitrogen and phosphorous content down; for "bio-remediation", i.e. the detoxification from heavy metals, dioxins, pesticides and PCBs of contaminated waters.

While at least one exemplary embodiment has been presented in the foregoing summary and detailed description, it should be appreciated that a vast number of variations exist. It should also be appreciated that the exemplary embodiment or exemplary embodiments are only examples, and are not intended to limit the scope, applicability, or configuration in any way. Rather, the foregoing summary and detailed description will provide those skilled in the art with a convenient road map for implementing at least one exemplary embodiment, it being understood that various changes may be made in the function and arrangement of elements described in an exemplary embodiment without departing from the scope as set forth in the appended claims and their legal equivalents.

### EXAMPLE 1: Stimulation of photoprotective antioxidant xanthophylls synthesis in a diatom species by light manipulation with the present system

### Experiments description

Four spectral light conditions - blue, red and two mixed light conditions, namely blue-red-green and blue-red were applied (see Table I). The two mixed light conditions were characterized by (i) the same photon flux density (PFD) and the same relative proportion of red radiation provided (25%), and (ii) two different red : blue ratios, for the blue-red-green mix the ratio is 0.50 while for the blue-red mix, the ratio is 0.33. Two daily light doses (sinusoidal light distribution, peaking at 200 µmol photons m⁻²s⁻¹ (low) and 500 µmol photons m⁻² s⁻¹ (high), see Table I) have been applied with a 12:12 hours light:dark photoperiod. For each condition, the daily light dose was kept constant, in order to be comparable for the provided photon flux density.

Experiments were conducted on the diatom *Skeletonema marinoi* (see Dimier et al., 2007 for more information on the species). Cells were cultivated at 20° C in 150 cm² polystyrene canted neck flasks (Corning® flask, Corning Inc., NY, USA), containing natural sterile seawater amended with f/2 nutrients. All the experiments lasted for two days and were performed in triplicate during the exponential growth phase of the cultures. Experiments were carried out on cultures pre-acclimated to each experimental light condition for two weeks before the experiments. Pigment measurement was conducted by High Performance Liquid Chromatography (HPLC) following the procedure described in Giovagnetti et al. (2012).

The results are presented in Table I:

**Table I. Experimental light properties and pigment content in Skeletonema marinoi**

| | **Blue** | | **Blue-red** | | **Blue-red-green** | | **Red** |
|---|---|---|---|---|---|---|---|
| | **Low** | **High** | **Low** | **High** | **Low** | **High** | **Low** |
| PFD | 200 | 500 | 200 | 500 | 200 | 500 | 200 |
| Blue | 200 | 500 | 150 | 375 | 100 | 250 | 0 |
| Green | 0 | 0 | 0 | 0 | 50 | 125 | 0 |
| Red | 0 | 0 | 50 | 125 | 50 | 125 | 200 |
| Red: Blue | 0 | 0 | 0.33 | 0.33 | 0.50 | 0.50 | 0 |
| | | | | | | | |
| Growth rate | 1.2 (0.10) | 0.8 (0.05) | 0.8 (0.04) | 0.6 (0.05) | 0.4(0.02) | 0.6(0.02) | 0.2 (0.06) |
| | | | | | | | |
| Ch1.*a* | 0.48 (0.15) | 0.42 (0.12) | 0.59 (0.15) | 0.44(0.11) | 0.45 (0.12) | 0.52 (0.06) | 0.52 (0.10) |
| | | | | | | | |
| Dd+Dt | 0.02 (0.005) | 0.08 (0.006) | 0.09 (0.005) | 0.04 (0.003) | 0.02 (0.005) | 0.10 (0.006) | 0.02 (0.003) |
| | | | | | | | |
| Dt/(Dt+Dd) | 0.06 (0.005) | 0.12 (0.005) | 0.10 (0.004) | 0.24 (0.006) | 0.11 (0.003) | 0.30 (0.008) | 0.0 (0.00) |

| | | | | | | | |
|---|---|---|---|---|---|---|---|
| Blue, green and red fluence rates (PFD, photon flux density, µmol photon m⁻² s⁻¹) were measured at light peak. Chl.a : chlorophyll *a* ; Dd: diadinoxanhtin ; Dt: diatoxanthin. Unit: pg cell⁻¹. Growth rate (per day, d⁻¹). Data represents mean and standard deviation (*n* = 21). Values in parentheses are Sd values. DES is de-epoxydation step and is the ratio Dt/(Dt+Dd). | | | | | | | |

### Results

The present study shows that spectral composition of light can modify the growth rate of cells (Table I). Indeed, cell growth rate is higher under low blue light (1.2) than under the other light conditions tested.

In addition, the spectral light composition is an essential trigger for photophysiological acclimation of diatoms, without compromising cell growth rate or chlorophyll *a* content (Table I). Chlorophyll a is the principal photosynthetic pigment in photosynthetic organisms.

The authors' results show that the fast photoprotective process such as the xanthophyll cycle activation (Dd and Dt synthesis and de-epoxydation step (DES), requires red light to be initiated and a high blue fluence rate to be activated. Furthermore, the red : blue ratio is a crucial parameter for modifying the photophysiological properties of the cells, mainly linked to pigment content related to light-harvesting complexes structures.

The relevance of this result concerns its potential application in algal biotechnology. Indeed, the xanthophyll cycle pigments (Dd: diadinoxanhtin and mainly diatoxanthin, Dt) might have an antioxidant role, countering peroxidative damages in cells (Lepetit et al., 2010; Brunet et al., 2011). The light system of the present invention is capable of inducing the synthesis of such molecules without decreasing biomass concentration or production efficiency from algae. This is due to the possibility of varying the spectral light composition together with the possibility of varying light quantity and light shape distribution. In the present system, sinusoidal distribution of light is generated, which is a much less stressful condition for the cells when compared to quadratic light distribution of existing light systems.

### BIBLIOGRAPHY

- Corato F., Brunet C., and Dimier C., 2007. Sviluppo ed applicazioni in fotobiologia algale di un sistema ad illuminazione variabile. Biologi Italiani, 2/2007: 37-43.
- Dimier C., Saviello G., Tramontano F. and Brunet C. 2009. Comparative ecophysiology of the xanthophyll cycle in six marine phytoplanktonic species. Protist, 160: 397-411.
- Dimier, C., Brunet C., Geider R. and Raven J., 2009. Growth and photoregulation dynamics of the picoeukaryote Pelagomonas calceolata in fluctuating light. Limnology and Oceanography, 54(3): 823-836.
- Brunet C, Johnsen G, Lavaud J, Roy S (2011) Pigments and photoacclimation processes. In Roy S, Llewellyn C, Skarstad Egeland E, Johnsen G editors. Phytoplankton Pigments, Characterization, Chemotaxonomy and Application in Oceanography. pp. 445-471.
- Dimier C., Corato F., Tramontano F. and Brunet C., 2007. Photoprotection and xanthophyll cycle activity in three diatoms. J. Phycol., 43(5): 937-947.
- Giovagnetti V., Cataldo M.L., Conversano F. and Brunet C., 2012. Growth and photophysiological response curves of the two picoplanktonic Minutocellus sp. RCC967 and RCC703 (Bacillariophyceae). Eur. J. Phycology. 47:4, 408-420.
- Lepetit B, Volke D, Gilbert M, Wilhelm C, Goss R (2010) Evidence for the existence of one antenna-associated, lipid-dissolved, and two protein-bound pools of diadinoxanthin cycle pigments in diatoms. Plant Physiol 154: 1905-1920.

## Claims

1. A light system (1) for aquatic photosynthetic organisms comprising:
- at least one panel (2), having a plurality of light emitting diode, LED, sensors, said LED sensors being independent of each other and being RGB LED sensors for providing a three color light comprising blue, red and green;
- three microchips coupled to the LED sensors for modifying the light color emitted by each LED sensor;
- an electronic device (3), said electronic device being connected to said at least one panel (2); and
- a computer (4)
said light system being **characterized in that** it is configured to combine the three colors with each other to obtain a wide range of different colors in the visible spectrum and wherein the light intensity of each color is independently between 0 and 600 µmol.m⁻² .s⁻¹.

2. The light system according to claim 1, wherein the size of said panel (2) has the same size as the surface to be illuminated.

3. The light system according to claim 1 or 2, wherein the size of said panel (2) ranges between 10 cm to 100 m in length and/or in height.

4. The light system according to any one of previous claims, wherein each panel (2) provides a light intensity reaching a maximum value of 1800 µmol.m⁻² .s⁻¹.

5. The light system according to any one of previous claims, wherein the light shape distribution is sinusoidal with high frequency time variations of light intensity and spectral composition ranging from seconds till hours to perform circadian cycles.

6. The light system according to any one of previous claims, wherein light intensity and/or light color may be varied in time by the user.

7. The light system according to any of the preceding claims, wherein said electronic device (3) comprises:
- a microprocessor (5), which interacts with the computer (4);
- an "EEPROM card" (6) for storing the data used for the setting of the system;
- an optoisolator (7) that allows to transfer a signal between two circuits while maintaining isolation between them using an optical system;
- an output circuit (8) with at least one transistor and
- a power supply board (9).

8. A bioreactor comprising the light system according to any one of previous claims.

9. Use of the light system according to any one of claims 1 to 7 or of the bioreactor according to claim 8 for the production of biomass, for the production of biomolecules, for the production of biofuel and/or biogas.

10. Use of the light system according to any one of claims 1 to 7 in a bioremediation method.

11. A method of controlling in real time the light system according to any of claims 1 to 7, **characterized by** the following steps:
a. initializing system parameters;
b. determining a photoperiod (light period vs. dark period);
c. sending of the signal to the light system;
d. light setting;
e. controlling the correct light setting (if ok, continue to step f, else go back to step b);
f. enabling a time counter to execute step g;
g. reading the light setting file;
h. controlling the time counter (if overcomes a maximum threshold continue to step i, else go back to step g)
i. checking execution and control steps (if ok continue to step j, else go back to step b);
j. ending the program.

12. A computer program comprising a computer-code suitable for performing the method according to claim 11.

13. A computer program product on which the computer program according to claim 12 is stored.

## Patentansprüche

1. Lichtsystem (1) für photosynthetische Wasserorganismen, umfassend:
- mindestens eine Platte (2), die mehrere Sensoren von Leuchtdioden (LED) aufweist, wobei die LED-Sensoren unabhängig voneinander sind und dreifarbiges Licht, umfassend blau, rot und grün, bereitstellen;
- drei Mikrochips, die mit den LED-Sensoren gekoppelt sind, um die Lichtfarbe zu modifizieren, die von jedem LED-Sensor emittiert wird;
- eine elektronische Vorrichtung (3), wobei die elektronische Vorrichtung mit der mindestens einen Platte (2) verbunden ist; und
- einen Computer (4),
wobei das Lichtsystem **dadurch gekennzeichnet ist, dass** es konfiguriert ist, um die drei Farben miteinander zu kombinieren, um einen weiten Bereich verschiedener Farben im sichtbaren Spektrum zu erhalten, und wobei die Lichtintensität jeder Farbe unabhängig zwischen 0 und 600 µmol.m⁻².s⁻¹ liegt

2. Lichtsystem nach Anspruch 1, wobei die Größe der Platte (2) die gleiche Größe wie die zu beleuchtende Oberfläche aufweist.

3. Lichtsystem nach Anspruch 1 oder 2, wobei die Größe der Platte (2) zwischen 10 cm bis 100 m Länge und/oder Höhe liegt.

4. Lichtsystem nach einem der vorhergehenden Ansprüche, wobei jede Platte (2) eine Lichtintensität aufweist, die einen maximalen Wert von 1.800 µmol.m⁻².s⁻¹ erreicht.

5. Lichtsystem nach einem der vorhergehenden Ansprüche, wobei die Verteilung der Lichtform sinusoidal ist mit hochfrequenten zeitlichen Variationen der Lichtintensität und Spektralzusammensetzung im Bereich von Sekunden bis Stunden, um zirkadiane Zyklen auszuführen.

6. Lichtsystem nach einem der vorhergehenden Ansprüche, wobei die Lichtintensität und/oder Lichtfarbe vom Benutzer zeitlich variiert werden kann.

7. Lichtsystem nach einem der vorhergehenden Ansprüche, wobei die elektronische Vorrichtung (3) umfasst:
- einen Mikroprozessor (5), der mit dem Computer (4) interagiert;
- eine "EEPROM-Karte" (6) zum Speichern der für die Einstellung des Systems verwendeten Daten;
- einen Optokoppler (7), der es ermöglicht, ein Signal zwischen zwei Schaltkreisen zu übertragen, während die Isolation zwischen ihnen unter Verwendung eines optischen Systems aufrechterhalten wird;
- einen Ausgangsschaltkreis (8) mit mindestens einem Transistor und
- eine Netzteilplatine (9).

8. Bioreaktor, umfassend das Lichtsystem nach einem der vorhergehenden Ansprüche,

9. Verwendung des Lichtsystems nach einem der Ansprüche 1 bis 7 oder des Bioreaktors nach Anspruch 8 zur Herstellung von Biomasse, zur Herstellung von Biomolekülen, zur Herstellung von Biokraftstoff und/oder Biogas.

10. Verwendung des Lichtsystems nach einem der Ansprüche 1 bis 7 in einem Bioremediationsverfahren.

11. Verfahren zum Steuern des Lichtsystems nach einem der Ansprüche 1 bis 7 in Echtzeit,
das durch die folgenden Schritte gekennzeichnet ist:
a. Initialisieren von Systemparametern;
b. Bestimmen einer Photoperiode (helle Periode im Verhältnis zu dunkler Periode);
c. Senden des Signals an das Lichtsystem;
d. Einstellen des Lichts;
e. Steuern der richtigen Lichteinstellung (wenn ok, weiter mit Schritt f, andernfalls zurück zu Schritt b);
f. Aktivieren eines Zeitzählers zum Ausführen von Schritt g;
g. Lesen der Lichteinstellungsdatei;
h. Steuern des Zeitzählers (wenn ein maximaler Schwellenwert überschritten wird, weiter mit i, andernfalls zurück zu Schritt g)
i. Überprüfen der Ausführungs- und Steuerschritte (wenn ok, weiter mit Schritt j, andernfalls zurück zu Schritt b);
j. Beenden des Programms.

12. Computerprogramm, umfassend einem Computercode, der zur Durchführung des Verfahrens nach Anspruch 11 geeignet ist.

13. Computerprogrammprodukt, auf dem das Computerprogramm nach Anspruch 12 gespeichert ist.

## Revendications

1. Système lumineux (1) pour organismes photosynthétiques aquatiques comprenant :
- au moins un panneau (2) ayant une pluralité de capteurs à diode électroluminescente (DEL), lesdits capteurs à DEL étant indépendants les uns des autres et étant des capteurs à DEL RVB pour fournir une lumière à trois couleurs comprenant le bleu, le rouge et le vert ;
- trois micropuces couplées aux capteurs à DEL pour modifier la couleur de lumière émise par chaque capteur à DEL ;
- un dispositif électronique (3), ledit dispositif électronique étant raccordé au dit au moins un panneau (2) ; et
- un ordinateur (4)
ledit système lumineux étant **caractérisé en ce qu'**il est configuré pour combiner les trois couleurs les unes avec les autres pour obtenir une vaste plage de couleurs différentes dans le spectre visible et dans lequel l'intensité de lumière de chaque couleur est indépendamment entre 0 et 600 µmol.m⁻².s⁻¹.

2. Système lumineux selon la revendication 1, dans lequel la taille dudit panneau (2) est la même que celle de la surface à éclairer.

3. Système lumineux selon la revendication 1 ou 2, dans lequel la taille dudit panneau (2) se trouve dans la plage comprise entre 10 cm et 100 m de longueur et/ou de hauteur.

4. Système lumineux selon l'une quelconque des revendications précédentes, dans lequel chaque panneau (2) fournit une intensité lumineuse atteignant une valeur maximale de 1 800 µmol.m⁻².s⁻¹.

5. Système lumineux selon l'une quelconque des revendications précédentes, dans lequel la distribution de la forme de la lumière est sinusoïdale avec des variations temporelles haute fréquence de l'intensité lumineuse et la composition spectrale dans la plage allant des secondes aux heures pour réaliser des cycles circadiens.

6. Système lumineux selon l'une quelconque des revendications précédentes, dans lequel l'intensité lumineuse et/ou la couleur de la lumière peuvent être modifiées dans le temps par l'utilisateur.

7. Système lumineux selon l'une quelconque des revendications précédentes, dans lequel ledit dispositif électronique (3) comprend :
- un microprocesseur (5), qui interagit avec l'ordinateur (4) ;
- une « carte EEPROM » (6) pour stocker les données utilisées pour la définition du système ;
- un optoisolateur (7) qui permet le transfert d'un signal entre deux circuits tout en maintenant l'isolation entre eux à l'aide d'un système optique ;
- un circuit de sortie (8) avec au moins un transistor et
- une carte d'alimentation (9).

8. Bioréacteur comprenant le système lumineux selon l'une quelconque des revendications précédentes.

9. Utilisation du système lumineux selon l'une quelconque des revendications 1 à 7 ou du bioréacteur selon la revendication 8 pour la production de biomasse, pour la production de biomolécules, pour la production de biocarburant et/ou de biogaz.

10. Utilisation du système lumineux selon l'une quelconque des revendications 1 à 7 dans un procédé de bioremédiation.

11. Procédé de commande en temps réel du système lumineux selon l'une quelconque des revendications 1 à 7, **caractérisé par** les étapes suivantes :
a. initialiser les paramètres du système ;
b. déterminer une photopériode (période de luminosité vs période d'obscurité) ;
c. envoyer le signal au système lumineux ;
d. régler la lumière ;
e. commander le réglage correct de la lumière (si ok, passer à l'étape f, dans le cas contraire revenir à l'étape b) ;
f. permettre à un compteur de temps d'exécuter l'étape g ;
g. lire le fichier de réglage de lumière ;
h. commander le compteur de temps (s'il dépasse un seuil maximal, passer à l'étape i, dans le cas contraire revenir à l'étape g) ;
i. vérifier les étapes d'exécution et de commande (si ok, passer à l'étape j, dans le cas contraire revenir à l'étape b) ;
j. terminer le programme.

12. Programme informatique comprenant un code informatique adapté à réaliser le procédé selon la revendication 11.

13. Produit de programme informatique sur lequel le programme informatique selon la revendication 12 est stocké.
